# EUROPEAN PATENT SPECIFICATION

(11) **EP 3 981 391 B1**
(45) Date of publication and mention of the grant of the patent: **30.10.2024**
(21) Application number: 20817973.9
(22) Date of filing: 27.05.2020
(51) Int. Cl.: A61K 36/258, A61K 9/16, A61K 9/48, A61K 9/20

(54) **ORAL PREPARATION HAVING IMPROVED DISSOLUTION RATE AND DISINTEGRATION PROPERTIES FOR NATURAL SUBSTANCE EXTRACT**
ORALE ZUBEREITUNG MIT VERBESSERTER AUFLÖSEGESCHWINDIGKEIT UND ZERFALLSEIGENSCHAFTEN FÜR NATURSTOFFEXTRAKT
PRÉPARATION ORALE AYANT UN TAUX DE DISSOLUTION ET DES PROPRIÉTÉS DE DÉSINTÉGRATION AMÉLIORÉS POUR UN EXTRAIT DE SUBSTANCE NATURELLE

(30) Priority: 04.06.2019 KR 20190066237
(43) Date of publication of application: 13.04.2022
(73) Proprietor: Green Cross WellBeing Corporation, Seoul 07335 (KR)
(72) Inventor: LIM, Nak Hyun, Seoul 07335 (KR); KIM, Jeom Yong, Seoul 07335 (KR); YOO, Young Hyo, Seoul 07335 (KR); PARK, Sun Kyu, Seoul 07335 (KR)
(74) Representative: Kraus & Lederer PartGmbB
(86) International application number: PCT/KR2020/006843
(87) International publication number: WO 2020/246744

(56) References cited:
- CN-A- 101 543 518
- CN-A- 105 853 366
- KR-A- 20080 034 154
- KR-A- 20100 022 711
- KR-A- 20120 138 521
- KR-A- 20130 027 822
- KR-A- 20150 095 708
- KR-B1- 101 966 117
- KR-B1- 102 095 536

## Description

### Technical Field

The present invention relates to an oral preparation having an improved dissolution rate and disintegrability of a natural product extract, and to an oral preparation having an improved dissolution rate and disintegrability of a natural product extract, such as a processed ginseng extract, obtained from a natural product, to increase bioavailability of the natural product extract.

### Background Art

In recent years, the paradigm of drug development has rapidly changed throughout the world. Adult diseases and chronic diseases are increasing due to the aging of the population, and the disease prevention concept for improving the quality of life is also growing in the pharmaceutical market, so that traditional medicines, of which clinical experience and safety are proved, and natural product medicines based thereon are expected to greatly grow in the suppression of aging including chronic diseases and the prevention and treatment of cancer, chronic diseases, adult diseases, and the like.

A natural product collectively refers to plants, animals, minerals, microorganisms, and metabolites thereof, obtained from the natural world, in a broad sense, and a new natural product drug refers to a medicine that is researched and developed using natural ingredients and has new constituent ingredients and effects. Industries using natural products are fast growing in developed countries, such as US, European countries, and Australia, due to increased interest and support on complementary and alternative medicines.

The natural product medicines need to have appropriate formulations in consideration of therapeutic effects, storage, and the like thereof. For example, the development of health supplements, functional cosmetics, and the like through accurate quality assurance while carrying out commercialization using formulations, such as tablets and capsules can greatly contribute to natural product industries. However, there is difficulty in controlling the dissolution of a natural product extract due to properties of the natural product extract.

A ginseng extract is one of the most representative natural product extracts. Ginsenosides, which are main functional ingredients of ginseng, are distinctively named only ginseng saponins among various types of saponins in the plant system, and at least 150 types of ginsenosides have been discovered so far. It has been revealed that ginsenosides have pharmacological effects, such as central nervous inhibition, mental tranquilization, pain relief, memory improvement, liver injury recovery, protein and lipid synthesis stimulation, anti-diabetes, anti-stress, antioxidant active substance production stimulation, immune regulation, platelet aggregation inhibition, and anti-aging actions, as well as anticancer, anti-allergy, anti-inflammation, wherein ginsenosides show respective different effects depending on the type thereof.

It has been known that: ginsenosides Rg1 and Rb1 enhance central nervous system activity; ginsenosides Re and Rg1 and panaxans A and B are good for diabetes; ginsenosides Re and Rg1 promote angiogenesis; and ginsenosides Rg3 and Rh2 exhibit anticancer effects. Recent studies revealed that some ginseng extracts show effects on cachexia, fatigue, or muscular atrophy. It was shown that GINSELECT^{®} containing 0.9-1.4% of ginsenoside Rg1 and 1.7-3.0% of ginsenoside Rb1 prevented the body weight reduction and motor ability reduction in mice treated with the anticancer tumor cisplatin (Lobina, C., et al., 2014) and ginsenoside Rg1 prevented muscular atrophy resulting from famine by inhibiting protein degradation pathways.

Ginsenosides are in an inactive form, and are known to have little physiological activity since they are highly soluble in water but are not well absorbed into the intestine. Active ginsenosides converted from the inactive ginsenosides by the separation of sugar are not easily soluble in water but highly absorbed into the human body, and thus exert physiological effects.

Therefore, in order to exert excellent physiological effects of ginsenosides, there is a need of development of preparations with improved dissolution rates and disintegrability of ginsenosides in the human body by increasing the solubility of active ginsenosides.

The present inventors have manufactured processed ginseng extracts with increased ginsenosides Rg3 and Rh2, which are trace ginsenoside components, by treatment with saponin degrading enzymes and hydrolysis with organic acids, and identified that these processed ginseng extracts have cancer-related fatigue preventing and treating effects and myogenesis promoting effects, and registered a patent thereof (Korean Patent No. 1595426; and Korean Patent No. 1966117).

Therefore, while researching natural product medicines using natural product extracts, the present inventors manufactured a processed ginseng extract with increased contents of ginsenosides Rg3 and Rh2 having excellent physiological activity, such as cancer-related fatigue preventing and treating effects and myogenesis promoting effects, and identified that the ginsenosides Rg3 and Rh2, which are main active ingredients of the manufactured processed ginseng extract, had low solubility in solvents excluding organic solvents. Therefore, the present inventors identified the constitution of an oral preparation with an improved dissolution rate and disintegrability of a processed ginseng extract in the living body by increasing the solubility and dispersibility of the ginsenosides to improve excellent physiological effects of the processed ginseng extract, and identified that the dissolution rate and disintegrability of a natural product extract are also improved in oral preparations containing other natural product extracts besides the processed ginseng extract.

Korean Patent No. 1454066 corresponding to the conventional art discloses a technique of formulating an enzymatically treated ginseng extract by using a poloxamer, polyethylene glycol, silicon dioxide, magnesium stearate, anhydrous calcium hydrogen phosphate, lactose, and crosslinked polyvinylpyrrolidone, but does not specifically disclose the dissolution rate effect of the ginseng extract according to the compositional ratio of the constituent ingredients of the present invention. In addition, Korean Patent Publication No. 2008-0034154 discloses a method for manufacturing an oral disintegrating tablet containing ginsenosides Rg3, Rg2, and Rh1 as main ingredients and polyethylene glycol, magnesium stearate, lactose, and crosslinked polyvinylpyrrolidone as auxiliary ingredients, but disclose neither a poloxamer, silicon dioxide, and dicalcium phosphate of the present invention, nor the compositional ratio of the constituent ingredients and the resultant dissolution rate increasing effect.
KR101966117B1 discloses a composition for promoting muscle differentiation comprising processed ginseng extract and, more particularly, a composition for promoting muscle differentiation comprising a ginseng extract having an increased amount of ginsenoside having an effect of promoting muscle differentiation.
KR20080034154A discloses a pharmaceutical composition comprising secondary ginseng glycosides and a method for manufacturing the same.
CN101543518A discloses a Chinese medicine extract of Radix Ginseng stem and leaf, especially a specific extract of stem and leaf of the total sapogenins of Radix Ginseng. CN105853366B discloses a solid dispersion of Qingyang ginseng which is the root of Cynanchum otophyllum Scheid.

### Disclosure of Invention

### Technical Problem

An aspect of the present invention is to provide an oral preparation with an improved dissolution rate and disintegrability of a natural product extract.

### Solution to Problem

In accordance with an aspect of the present invention, there is provided an oral preparation containing a natural product extract and having an improved dissolution rate and disintegrability of the natural product extract, the oral preparation comprising a solubilizer, a water-insoluble diluent, a lubricant, and a disintegrant, wherein
i) the solubilizer is a mixture of a poloxamer and polyethylene glycol (PEG), the poloxamer and polyethylene glycol being mixed at a weight ratio of 1-10:1-10;
ii) the water-insoluble diluent is at least one selected from the group consisting of dicalcium phosphate, cellulose, and starch;
iii) the lubricant is at least one selected from the group consisting of silicon dioxide and magnesium stearate; and
iv) the disintegrant is at least one selected from the group consisting of crospovidone and sodium croscamellose, and
wherein the natural product extract is a processed ginseng extract or a ginseng extract.

The oral preparation may contain, relative to 100 parts by weight of the natural product extract, 30-120 parts by weight of the solubilizer, 20-100 parts by weight of the water-insoluble diluent, 3-20 parts by weight of the lubricant, and 5-20 parts by weight of the disintegrant.

The processed ginseng extract may be prepared by: (a) seeding Aspergillus niger into a medium composed of a ginseng powder and wheat bran; (b) culturing the fungus in step (a); (c) purifying the culture in step (b) through ultrafiltration; (d) separating an enzyme from the purified product in step (c); (e) adding the enzyme in step (d) to a ginseng powder, a red ginseng powder, a ginseng extract, or a red ginseng extract; (f) fermenting the addition product in step (e); (g) isolating the fermented product in step (f); (h) concentrating the supernatant in step (g); (i) reacting the concentrate in step (h) with at least one organic acid selected from the group consisting of acetic acid, lactic acid, citric acid, malic acid, and tartaric acid; and (j) neutralizing, filtering, purifying, concentrating, and drying the reaction product in step (i).

The solubilizer is a mixture of a poloxamer and polyethylene glycol (PEG), the poloxamer and polyethylene glycol being mixed at a weight ratio of 1-10:1-10.

The water-insoluble diluent may be at least one selected from the group consisting of dicalcium phosphate, cellulose, and starch.

The lubricant may be at least one selected from the group consisting of silicon dioxide and magnesium stearate.

In the mixture of silicon dioxide and magnesium stearate, silicon dioxide and magnesium stearate may be mixed at a weight ratio of 1-5:0.1-1.

The disintegrant may be at least one selected from the group consisting of crospovidone and sodium croscamellose.

The oral preparation may further contain a water-soluble diluent.

The water-soluble diluent may be at least one selected from the group consisting of lactose, sucrose, mannitol, sorbitol, and dextrin.

The water-soluble diluent may be mixed with the water-insoluble diluent at a weight ratio of 1:0.2-5.

The oral preparation may contain a poloxamer as the solubilizer and dicalcium phosphate as the water-insoluble diluent.

Hereinafter, the present invention will be described in detail.

The present invention is directed to an oral preparation containing a natural product extract and including a solubilizer, a water-insoluble diluent, a lubricant, and a disintegrant as defined above.

The term "natural product" collectively refers to plants, animals, minerals, microorganisms, and metabolites thereof, obtained from the natural world. Most of the extracts extracted from natural products have low solubility in water, and thus the dissolution rates of the extracts are difficult to control in the development to medicines, health functional foods, and cosmetic products, and the like.

A natural product extract has various physiological activities applicable to medicines, health functional foods, and cosmetic products, and may have low solubility in a water-soluble solvent such as water, unfavorable wettability when dispersed in a solvent, or have low dispersibility and solubility due to the aggregation thereof by a solvent.

The plant extract may be an extract that is obtained by further applying enzymes, acid-base, and physical factors to an extract extracted from a plant, a fraction obtained by fractionation of an extract with an organic solvent, or a fraction obtained through column chromatography of an extract. However, the plant extract is not limited thereto.

The extract of a plant of the family Araliaceae is an extract extracted from a plant of the family Araliaceae, and the plant of the family Araliaceae is ginseng, *Eleutherococcus sessiliflorus, Kalopanax pictus, Dendropanax morbiferus,* or the like. The ginseng extract has been well known to have saponins, ginsenosides, and the like as active ingredients, and there are various pharmacological reports for those ingredients, and especially, those ingredients show excellent effects of preventing cancer, inhibiting cancer cell metastasis, inhibiting fatigue, inhibiting platelet aggregation, and promoting muscular cell differentiation.

The ginseng extract is an extract extracted from ginseng or processed ginseng. The ginseng extract may be an extract obtained by further treating, with enzymes or acid-base, an extract extracted from ginseng or processed ginseng, a fraction obtained by fractionating, with organic solvents, an extract extracted from ginseng or processed ginseng, or a fraction obtained through column chromatography of the extract. However, the ginseng extract is not limited thereto.

The ginseng extract may be prepared by the preparation methods of Korean Patent Nos. 0992800, 1595426, and 1966117, and may be a processed ginseng powder or a processed ginseng extract with increased trace ginsenoside components through treatment with saponin degrading enzymes and hydrolysis with organic acids.

Specifically, the processed ginseng powder or processed ginseng extract may be a processed ginseng powder or processed ginseng extract with increased trace ginsenoside components, which is prepared by (a) seeding *Aspergillus niger* into a medium composed of a ginseng powder and wheat bran; (b) culturing the fungus in step (a); (c) purifying the culture in step (b) through ultrafiltration; (d) separating an enzyme from the purified product in step (c); (e) adding the enzyme in step (d) to a ginseng powder, a red ginseng powder, a ginseng extract, or a red ginseng extract; (f) fermenting the addition product in step (e); (g) isolating the fermented product in step (f); (h) concentrating the supernatant in step (g); (i) reacting the concentrate in step (h) with at least one organic acid selected from the group consisting of acetic acid, lactic acid, citric acid, malic acid, and tartaric acid; and (j) neutralizing, filtering, purifying, concentrating, and drying the reaction product in step (i).

The trace ginsenosides may be ginsenosides having excellent physiological effects, and may be preferably ginsenosides Rg3 and Rh2.

In the processed ginseng powder or processed ginseng extract, the content of each of ginsenosides Rg3 and Rh2 may be 0.2-30 wt%, preferably 0.5-30 wt%, and more preferably 1-20 wt%. The processed ginseng powder or processed ginseng extract may be most preferably a processed ginseng powder or processed ginseng extract containing 13 wt% of ginsenoside Rg3 and 7 wt% of ginsenoside Rh2.

The processed ginseng powder or processed ginseng extract has cancer-related fatigue preventing or treating effects as well as myogenesis promoting and muscle atrophy inhibiting effects, and thus among the oral preparations containing the natural product extract of the present invention, an oral preparation containing the processed ginseng powder or processed ginseng extract can be used to treat various types of diseases, such as cachexia, sarcopenia, and muscle atrophy.

Ginsenosides Rg3 and Rh2, which are active ingredients in the processed ginseng powder or processed ginseng extract, have high solubility in organic solvents, such as isopropanol and ethanol, but have remarkably low solubility in water or dissolution media disclosed in the Korean Pharmacopoeia. Specifically, ginsenosides Rg3 and Rh2 show a solubility of less than 0.3 mg/g in water, a pH 1.2 buffer, or a pH 4.0 buffer. In addition, the processed ginseng powder or processed ginseng extract is not well wetted but agglomerated in the dissolution media and thus show low dispersibility.

The oral preparation of the present invention can increase the bioavailability of the natural product extract by increasing the solubility and dispersibility of the natural product extract to improve the dissolution rate and disintegrability, in the human body, of the natural product extract of the oral preparation containing the natural product extract.

In the oral preparation containing the natural product extract compared with a preparation containing only the natural product extract alone, the dissolution rate of the natural product extract is increased by 2.5 times or more, and the natural product extract may be disintegrated within 5 minutes.

The oral preparation containing the natural product extract as defined above may contain, relative to 100 parts by weight of the natural product extract, 30-120 parts by weight of a solubilizer, 20-100 parts by weight of a water-insoluble diluent, 3-20 parts by weight of a lubricant, and 5-20 parts by weight of a disintegrant. Preferably, the oral preparation may contain, relative to 100 parts by weight of the natural product extract, 50-100 parts by weight of a solubilizer, 30-100 parts by weight of a water-insoluble diluent, 5-15 parts by weight of a lubricant, and 6-18 parts by weight of a disintegrant, and more preferably, relative to 100 parts by weight of the natural product extract, 60-90 parts by weight of a solubilizer, 40-90 parts by weight of a water-insoluble diluent, 7-15 parts by weight of a lubricant, and 8-16 parts by weight of a disintegrant. Most preferably, the oral preparation contains, relative to 100 parts by weight of the natural product extract, 65-75 parts by weight of a solubilizer, 50-80 parts by weight of a water-insoluble diluent, 7-11 parts by weight of a lubricant, and 10-15 parts by weight of a disintegrant.

In the oral preparation containing the natural product extract, the natural product extract may be contained in 80-120 mg/capsules. Preferably, 100 mg/capsules may be contained. A content of the natural product extract outside the above range is undesirable since such a content may result in the administration of a specific active ingredient in a small or excessive amount, thereby producing different effects.

In the oral preparation of the present invention, among the solubilizers usable to improve the solubility of natural product extracts, a material that is commonly used in oral preparations, such as a tablet, a capsule, and a liquid preparation, may be used as a solubilizer.

The solubilizer is a mixture of a poloxamer and polyethylene glycol at a weight ratio of 1-10:1-10. Preferably, the poloxamer and the polyethylene glycol may be mixed at a weight ratio of 5-10:1-5.

In the present invention, "poloxamer (pluronic)" is a non-ionic surfactant and is a polymer of poly(oxyethylene)-poly(oxypropylene)-poly(oxyethylene) (POE-POP-POE). It has been reported that poloxamer solutions are liquid at low temperatures but have viscosity increasing with increasing temperature, show a sol-gel phase change over temperature at high concentrations, and do not damage mucosal cell membranes. Poloxamers are widely used as solubilizers, emulsifiers, wetting agents, and the like in the pharmaceutical industry, and the range of use thereof is expanding, such as a sustained preparation for sustained release of a drug at a burnt site, an ophthalmic solution, an injection, and the like.

The poloxamer is an additive with high self-solubility. The poloxamer is present between powders and dissolves quickly upon dissolution, so that the solution forms a channel between the additives to separate the natural product extract from the other additives, thereby improving dispersibility. In addition, the poloxamer is a hydrophilic non-ionic surfactant, and acts as a solubilizer in a solution to increase the solubility of the natural product extract with low solubility, thereby improving the dissolution rate.

The poloxamer may be any poloxamer usable in the art. Preferably, the poloxamer may be at least one selected from the group consisting of Poloxamer 188 and Poloxamer 407.

The poloxamers of the present invention may be classified into a micronized type and a granular type according to particle size. The poloxamers as the solubilizers may employ a micronized type or a granular type alone or a mixture of a micronized type and a granular type, and a micronized type having a small particle size is preferable.

The poloxamer may be contained in 30-120 parts by weight relative to 100 parts by weight of the natural product extract. The poloxamer may be contained in preferably 40-100 parts by weight, and more preferably 50-80 parts by weight. When the poloxamer is contained in less than 30 parts by weight or more than 120 parts by weight, the dissolution rate or disintegrability of the natural product extract deteriorates and the stability of the natural product extract against heat or moisture retention is not ensured, and thus such contents are not preferable.

In the present invention, "polyethylene glycol (PEG)" is a non-ionic surfactant and may be obtained by combining ethylene oxide. Polyethylene glycol may be used as a solvent, a surfactant, an emulsifier, a stabilizer, a lubricant, or the like. In the pharmaceutical industry, the polyethylene glycol series is used as excipients or adjuvants.

The polyethylene glycol may be any polyethylene glycol usable in the art. Preferably, the polyethylene glycol has a molecular weight of 200-6000. Polyethylene glycol having a molecular weight of more than 6000 may be toxic and thus is not preferable.

The polyethylene glycol may be mixed with a poloxamer to quickly increase the dissolution rate of the natural product extract.

In the oral preparation, the solubilizer may be contained in 30-120 parts by weight relative to 100 parts by weight of the natural product extract. When the solubilizer is contained in less than 30 parts by weight or more than 120 parts by weight, the disintegrability and dissolution rate of the natural product extract may be lowered, and thus such contents are not preferable.

In the oral preparation, the water-insoluble diluent has remarkably low solubility in water and can weaken the cohesive force of the natural product extract to improve the dispersibility thereof. As the content of the water-insoluble diluent increases, the disintegration time of the natural product extract may be advanced, thereby affecting the dissolution rate of the natural product extract.

The water-insoluble diluent is at least one selected from the group consisting of dicalcium phosphate, cellulose and starch. More preferably, the water-soluble diluent may be at least one selected from the group consisting of dicalcium phosphate and cellulose. Most preferably, the water-soluble diluent is dicalcium phosphate.

In the present invention, as a result of investigating the dissolution rate and disintegrability of the natural product extract after an oral preparation was prepared using cellulose (microcrystalline cellulose) or starch except for dicalcium phosphate as a water-insoluble diluent, the same effect as when dicalcium phosphate was used was obtained.

The dicalcium phosphate may employ any dicalcium phosphate usable in the art. In some cases, dicalcium phosphate of hydrates may be further subjected to a pulverizing process before use due to a large particle size and a large volume to mass, and thus for the minimization of a process, it is preferable to use dicalcium phosphate of anhydrates having a small particle size and volume.

The water-insoluble diluent may be contained in 20-100 parts by weight relative to 100 parts by weight of the natural product extract. When the water-insoluble diluent is contained in less than 20 parts by weight or more than 100 parts by weight, the disintegration time may be delayed or the hardness of a tablet may be increased upon tableting, resulting in a decreased dissolution rate, and thus such contents are not preferable.

The oral preparation may further contain a water-soluble diluent.

The water-soluble diluent has high solubility in water, and a material that is commonly used pharmaceutically may be used within the range that does not affect drug efficacy, and examples thereof may be lactose, sucrose, mannitol, sorbitol, dextrin, and the like. The water-soluble diluent may be preferably at least one selected from the group consisting of lactose, sucrose, mannitol, sorbitol, and dextrin, and more preferably at least one selected from the group consisting of lactose and mannitol. Most preferably, the water-soluble diluent is lactose.

The water-soluble diluent may be mixed with a water-insoluble diluent, and the water-soluble diluent and the water-insoluble diluent may be mixed at a weight ratio of 1:0.2-5. Preferably, the water-soluble diluent and the water-insoluble diluent may be mixed at a weight ratio of 1:0.3-5.

In the oral preparation, the mixture of a water-soluble diluent and a water-insoluble diluent may be contained in 80-160 parts by weight relative to 100 parts by weight of the natural product extract. When the mixture of a water-soluble diluent and a water-insoluble diluent is contained in less than 80 parts by weight or more than 160 parts by weight, the fluidity and disintegration time of the natural product extract may be decreased and delayed, and thus such contents are not preferable.

In the oral preparation, a lubricant can prevent the attachment of a drug agent to a tableting punch during tableting and can increase the fluidity of granules. The lubricant is at least one selected from the group consisting of silicon dioxide and magnesium stearate.

As for the silicon dioxide, all the silicon dioxide compounds usable in the art may be used alone or in mixture. Preferably, colloidal silicon dioxide is used alone or in mixture. The colloidal silicon dioxide can reduce the volume compared with general silicon dioxide and can further improve fluidity, miscibility, and the like.

The mixture of silicon dioxide and magnesium stearate may be a mixture in which silicon dioxide and magnesium stearate are mixed at a weight ratio of 1-5:0.1-1. The silicon dioxide and magnesium stearate may be mixed at a weight ratio of preferably 1-4.5:0.5-1, and more preferably 2-4.5:1

In the oral preparation, the lubricant may be contained in 3-20 parts by weight relative to 100 parts by weight of the natural product extract. When the lubricant is contained in less than 3 parts by weight, the fluidity of a powder may be reduced, causing the deterioration in compression and dissolution of capsules or tablets, and thus such contents are not preferable. When the lubricant is contained in more than 20 parts by weight, the binding properties between powders may deteriorate and the disintegration may be delayed, and thus such contents are not preferable.

In the oral preparation, the disintegrant is used to increase the disintegration rate. The disintegrant is at least one selected from the group consisting of crospovidone and sodium croscamellose. The disintegrant may be preferably crospovidone.

In the oral preparation, the disintegrant may be contained in 5-20 parts by weight relative to 100 parts by weight of the natural product extract. A disintegrant contained in less than 5 parts by weight may delay the disintegration time and thus is not preferable, and a disintegrant contained in more than 20 parts by weight may affect the stability of a finished medicine due to madescent properties, and thus is not preferable.

A water-soluble diluent and a disintegrant are generally used in the art for a preparation with an improved dissolution rate and disintegrability of the natural product extract. However, a poloxamer as a solubilizer and dicalcium phosphate as a water-insoluble diluent may be used in order to improve the dissolution rate and disintegrability of the natural product extract of the present invention.

When the oral preparation containing the natural product extract of the present invention is tested at a temperature of 37.0±0.5°C in 900 ml of artificial gastric juice (containing 1% (w/v) Tween 80) eluate with pH of 1.2 at 50 rpm according to a dissolution test method (paddle method) disclosed in the Korean Pharmacopoeia, the dissolution rate of the natural product extract is 85% or more within 60 minutes and 75% or more within 45 minutes, and the preparation may be disintegrated within 5 minutes.

Even when the oral preparation containing the natural product extract of the present invention is stored for 3 months under long-term storage conditions of 25°C and relative humidity of 60% or under accelerated storage conditions of 40°C and relative humidity of 75%, the content of the natural product extract is maintained at 100±5% and the dissolution rate is maintained at 80-90%, so that the oral preparation shows excellent stability.

In the present invention, a ginseng extract as an extract of a plant of the family Araliaceae, besides the processed ginseng extract, were used as natural product extracts to manufacture oral preparations containing the respective plant extracts, and then the dissolution rate and disintegrability of each preparation were investigated while ginsenoside Rg3 was used as a reference substance for the extract of ginseng of the family Araliaceae, secoxyloganin was used as a reference substance for the Lonicerae Flos extract, linarin was used as a reference substance for the *Chrysanthemum zawadskii* extract, and platycodin was used as a reference substance for the balloonflower extract. As a result, it was identified that the dissolution rate and disintegrability of each plant extract in the oral preparations of the present invention were improved.

The oral preparation of the present invention may be variously prescribed by factors, such as the patient's disease type, disease severity, type of formulation, patient's age, sex, weight, health status, and diet, the administration time and administration method of a pharmaceutical composition for treatment, the administration route, excretion rate, and response sensitivity. The dose of the preparation of the present invention is preferably 0.001-100 mg/kg per day, more preferably 0.01-80 mg/kg, and most preferably 0.1-60 mg/kg, for adults. The preparation of the present invention may be administered once a day or divided into twice a day at regular time intervals according to the determination of a doctor or pharmacist, and preferably administered twice a day to improve the patient's drug compliance.

The oral preparation of the present invention may be a tablet, pills, a powder, granules, a capsule, or the like.

### Advantageous Effects of Invention

The present invention is directed to an oral preparation with an improved dissolution rate and disintegrability of a natural product extract, wherein a processed ginseng extract with increased ginsenoside Rg3 and Rh2, which are trace ginsenoside components, was used as representative natural product extracts, and it was identified that the processed ginseng extract contained a poloxamer as a solubilizer and a water-insoluble diluent as main ingredients and, besides, contained a lubricant, a water-soluble diluent, and a disintegrant, wherein the dissolution rate and disintegrability of the processed ginseng extract were improved in the oral preparation manufactured by the identified constitution, and the improved dissolution rate and disintegrability were maintained even upon the long-term storage.

Therefore, the oral preparation with an improved dissolution rate and disintegrability of the natural product extract of the present invention is expected to be usable in the development of an oral preparation with high physiological effect through increased bioavailability of the natural product extract.

### Brief Description of Drawings

FIG. 1 shows the results of identifying the dissolution rates of ginsenosides Rg3 (A) and Rh2 (B) of an oral preparation containing a processed ginseng extract according to the type of silicon dioxide used as a lubricant.
FIG. 2 shows the results of identifying the dissolution rates of ginsenosides Rg3 (A) and Rh2 (B) of an oral preparation containing a processed ginseng extract according to the content of dicalcium phosphate used as a water-insoluble diluent.
FIG. 3 shows the results of identifying the dissolution rates of ginsenosides Rg3 (A) and Rh2 (B) of an oral preparation containing a processed ginseng extract according to the mixing amount of dicalcium phosphate used as a water-insoluble diluent, lactose used as a water-soluble diluent, and silicon dioxide used as a lubricant.
FIG. 4 shows the results of identifying the dissolution rates of ginsenosides Rg3 (A) and Rh2 (B) of an oral preparation containing a processed ginseng extract according to the type of poloxamer used as a solubilizer.
FIG. 5 shows the results of identifying the dissolution rate of ginsenoside Rg3 of an oral preparation containing a processed ginseng extract according to the content of a poloxamer used as a solubilizer.
FIG. 6 shows the results of identifying the dissolution rates of ginsenosides Rg3 (A) and Rh2 (B) of an oral preparation containing a processed ginseng extract according to the mixing content of a poloxamer used as a solubilizer and silicon dioxide used as a lubricant and the type of silicon dioxide.
FIG. 7 shows the results of identifying the dissolution rate of ginsenoside Rg3 of an oral preparation containing a processed ginseng extract according to the addition or non-addition of polyethylene glycol (PEG) to a poloxamer used as a solubilizer.
FIG. 8 shows the results of identifying the dissolution rates of ginsenosides Rg3 (A) and Rh2 (B) of an oral preparation containing a processed ginseng extract according to whether a poloxamer used as a solubilizer and dicalcium phosphate used as a water-insoluble diluent are contained.
FIG. 9 shows the results of identifying dissolution rate and disintegrability of an oral preparation containing a processed ginseng extract of the present invention.
FIG. 10 shows the results of identifying the contents of ginsenosides Rg3 (A) and Rh2 (B) according to the storage condition of an oral preparation containing a processed ginseng extract of the present invention.
FIG. 11 shows the results of identifying the change in dissolution rate of ginsenoside Rg3 according to the storage condition of an oral preparation containing a processed ginseng extract of the present invention.

### Best Mode for Carrying out the Invention

Hereinafter, preferable exemplary embodiments of the present invention will be described in detail. However, the present invention is not limited to the exemplary embodiments described herein and can be embodied in many different forms.

### <Test Example 1: Solubility of processed ginseng extracts>

Processed ginseng extracts were used as natural product extracts of the present invention, and the processed ginseng extracts were prepared by the methods disclosed in Korean Patent Nos. 1595426 and 1966117. Among these, a processed ginseng extract containing 13 wt% of ginsenoside Rg3 and 7 wt% of ginsenoside Rh2 was used.

To investigate the solubility of the processed ginseng extract of the present invention, isopropanol, ethanol, water, and a pH 1.2 buffer, a pH 4.0 buffer, and a pH 6.8 buffer, which are dissolution media disclosed in the Korean Pharmacopoeia, were used as solvents. After 500 mg of the processed ginseng extract was added to 2 g of each solvent, stirring was performed at 50 rpm and 37°C for 18 hours, thereby obtaining each sample. Thereafter, a filtrate obtained by filtration of each sample through a 0.45-µm polyvinylidene fluoride (PVDF) syringe filter was analyzed by high performance liquid chromatography (HPLC) to investigate the solubility of ginsenosides Rg3 and Rh2 contained in the processed ginseng extract. The results are shown in Table 1 below.
- HPLC Conditions were as follows:
- Column: C18, 1.8 µm, 2.1x50 mm
- Column Temperature: 50°C
- Detector: UV 203 nm
- Flow rate: 0.6 ml/min
- Amount of injection: 2 µl
- Run time: About 14 min
- Mobile phase: distilled water: acetonitrile =57:43 (v/v)

**TABLE 1**

| Solvent | Ginsenoside Rg3 (mg/g) | | Ginsenoside Rh2 (mg/g) | |
|---|---|---|---|---|
| | S-form | R-form | S-form | R-form |
| Isopropanol | 7.43 | 4.72 | 3.99 | 2.47 |
| Ethanol | 7.52 | 5.45 | 3.96 | 2.55 |
| pH 1.2 buffer | 0.01 | 0.01 | 0.00 | 0.00 |
| pH 4.0 buffer | 0.28 | 0.05 | 0.13 | 0.00 |
| pH 6.8 buffer | 1.23 | 0.31 | 0.60 | 0.09 |
| Water | 0.21 | 0.05 | 0.10 | 0.00 |

As shown in Table 1 above, the solubility of ginsenosides Rg3 and Rh2 was high when isopropanol and ethanol were used as solvents, but in the use of the dissolution media disclosed in the Korean Pharmacopoeia, the ginsenosides were partially dissolved only in the pH 6.8 buffer but showed very low solubility in the pH 1.2 and pH 4.0 buffers. It was also visually observed that the processed ginseng extract was not well wetted but agglomerated in the dissolution media disclosed in the Korean Pharmacopoeia, indicating that the processed ginseng extract needed to improve solubility and dispersibility thereof.

### <Test Example 2: Additives for improving solubility and dispersibility of natural product extract>

### Test example 2-1: Appearance according to type of solubilizer

For the development of an oral preparation, such as a tablet or a capsule, with an improved dissolution rate and disintegrability of the natural product extract of the present invention, the appearance of a mixture according to the type of solubilizer was examined to select a solubilizer capable of increasing the solubility and dispersibility of the processed ginseng extract as a representative natural product extract.

Specifically, the processed ginseng extract was mixed with each solubilizer at a weight ratio therebetween shown in Table 2 below. In the case of the mixture containing polyethylene glycol 400 (PEG400), one of the solubilizers, the processed ginseng extract was first mixed with PEG400 and then additionally mixed with another solubilizer. Each mixture was warmed in a dryer at 60°C for 1 hour, stirred well such that there was no processed ginseng extract that was not wetted, and warmed at 60°C for additional 2 hours. The appearance of each mixture was visually examined, and the results are shown in Table 2 below.

**TABLE 2**

| Mixing ratio (weight ratio) | | | | | | | | | | | | | | | | Final appear ance |
|---|---|---|---|---|---|---|---|---|---|---|---|---|---|---|---|---|
| Proce ssed ginse ng extra ct | Solubilizer | | | | | | | | | | | | | | | |
| | PEG 200 | PEG 300 | PEG 400 | PEG 600 | PEG 4000 | PEG 6000 | Gelu cire 44/14 | Trans cutol P | Solu plus | TW 80 | Cremo phor EL | Cremo phor RH40 | Poloxa mer 188 | Poloxa mer 407 | Solu tol HS15 | |
| 1 | 1 | - | - | - | - | - | - | - | - | - | - | - | - | - | - | Viscou s materi al |
| 1 | - | 1 | - | - | - | - | - | - | - | - | - | - | - | - | - | Viscou s materi al |
| 1 | - | - | 1 | - | - | - | - | - | - | - | - | - | - | - | - | Viscou s materi al |
| 1 | - | - | - | 1 | - | - | - | - | - | - | - | - | - | - | - | Viscou s materi al |
| 1 | - | - | - | - | 1 | - | - | - | - | - | - | - | - | - | - | Hard solid |
| 1 | - | - | - | - | - | 1 | - | - | - | - | - | - | - | - | - | Hard solid |
| 1 | - | - | - | - | - | - | 1 | - | - | - | - | - | - | - | - | Semi-solid |
| 1 | - | - | - | - | - | - | - | 1 | - | - | - | - | - | - | - | Viscou s soluti on |
| 1 | - | - | - | - | - | - | - | - | 1 | - | - | - | - | - | - | Mixed state |
| 1 | - | - | - | - | - | - | - | - | - | 1 | - | - | - | - | - | Viscou s soluti on |
| 1 | - | - | - | - | - | - | - | - | - | - | 1 | - | - | - | - | Viscou s soluti on |
| 1 | - | - | - | - | - | - | - | - | - | - | - | 1 | - | - | - | Semi-solid |
| 1 | - | - | - | - | - | - | - | - | - | - | - | - | 1 | - | - | Solid |
| 1 | - | - | - | - | - | - | - | - | - | - | - | - | - | 1 | - | Solid |
| 1 | - | - | - | - | - | - | - | - | - | - | - | - | - | - | 1 | Semi-solid |
| 1 | - | - | 1 | - | - | - | 1 | - | - | - | - | - | - | - | - | Semi-solid |
| 1 | - | - | 1 | - | - | - | - | 1 | - | - | - | - | - | - | - | Viscou s soluti on |
| 1 | - | - | 1 | - | - | - | - | - | 1 | | - | - | - | - | - | Solid |
| 1 | - | - | 1 | - | - | - | - | - | - | 1 | - | - | - | - | - | Viscou s |
| | | | | | | | | | | | | | | | | soluti on |
| 1 | - | - | 1 | - | - | - | - | - | - | - | 1 | - | - | - | - | Viscou s soluti on |
| 1 | - | - | 1 | - | - | - | - | - | - | - | - | 1 | - | - | - | Solid |
| 1 | - | - | 1 | - | - | - | - | - | - | - | - | - | 1 | - | - | Solid |
| 1 | - | - | 1 | - | - | - | - | - | - | - | - | - | - | 1 | - | Solid |
| 1 | - | - | 1 | - | - | - | - | - | - | - | - | - | - | - | 1 | Semi-solid |
| PEG : Polyethylene glycol, TW80 : Tween80 | | | | | | | | | | | | | | | | |

As shown in Table 2 above, the appearance of the mixture of the processed ginseng extract and a solubilizer vary depending on the type of solubilizer. For solubilizers showing a solid or semi-solid appearance to be suitable for the manufacture of tablets and capsules among the solubilizers, the solubility and dispersibility of the processed ginseng extract were investigated. Solubilizers with high solubility upon visual observation were also used for tests even when the final appearances thereof were solutions.

### Test example 2-2: Solubility and dispersibility according to type of solubilizer

The solubility and dispersibility of the mixtures of the processed ginseng extract and the solubilizers identified in Test Example 2-1 were investigated, and the solubility and dispersibility of the mixtures in which some additives were further mixed besides the solubilizers were also investigated.

Specifically, the ingredients shown in Table 3 were mixed to obtain mixtures, and 300 mg of the obtained mixtures were filled in No. 1 capsules, respectively. The capsules filled with the mixtures were visually observed for solubility and dispersibility through a disintegration test in a disintegrator containing a pH 1.2 buffer (dissolution medium) at 37°C. The results are shown in Table 3 below.

**TABLE 3**

| Mixture | | | | | Result | | |
|---|---|---|---|---|---|---|---|
| Processed ginseng extract (mg) | Solubilizer | | Additive | | Solubi lity | Diaper sibili ty | Others |
| | Type | Additi on amount (mg) | Type | Additi on amount (mg) | | | |
| 500 | PEG4000 | 500 | - | - | Low | Low | - |
| 500 | PEG6000 | 500 | - | - | Low | Low | - |
| 500 | Poloxamer 188 | 500 | - | - | Low | Low | - |
| 500 | Poloxamer 407 | 500 | - | - | High | High | - |
| 500 | Gelucire 44/14 | 500 | PEG400 | 500 | High | High | - |
| 500 | Transcutol P | 500 | PEG400 | 500 | Medium | Medium | - |
| 500 | Soluplus | 500 | PEG400 | 500 | Low | Low | - |
| 500 | Tween80 | 500 | PEG400 | 500 | Medium -High | Medium -High | Microparticles being present |
| 500 | Cremophor EL | 500 | PEG400 | 500 | Medium -High | Medium -High | Microparticles being present |
| 500 | Cremophor RH40 | 500 | PEG400 | 500 | Low | Low | - |
| 500 | Poloxamer 188 | 500 | PEG400 | 500 | Medium -High | Medium -High | Microparticles being present |
| 500 | Poloxamer 407 | 500 | PEG400 | 500 | Medium -High | Medium -High | Microparticles being present |
| 500 | Solutol HS15 | 500 | PEG400 | 500 | Medium -High | Medium -High | Microparticles being present |
| 500 | Poloxamer 407 | 250 | Aerosil Pharma 200* | 50 | Low | Low | - |
| 500 | Poloxamer 407 | 250 | CaSiO₂ | 50 | High | High | - |
| 500 | Poloxamer 407 | 250 | Aerosil Pharma 300* | 50 | Medium | Medium | - |
| 500 | Poloxamer 407 | 350 | CaSiO₂ | 50 | High | High | - |
| 500 | Poloxamer 407 | 500 | CaSiO₂ | 50 | High | High | - |
| 500 | Poloxamer 407 | 350 | CaSiO₂ | 50 | High | High | - |
| * Aerosil Pharma 200, Aerosil Pharma 300 : silicon dioxide (SiO₂) | | | | | | | |

As shown in Table 3, the solubility and dispersibility of the mixtures were improved when Poloxamer 407 was used alone or Gelucire 44/14, Transcutol P, Tween80, Cremophor EL, Poloxamer 188, or Poloxamer 407 was mixed with SiO₂ (silicon dioxide) as an additive, such as PEG400, CaSiO₂ (calcium silicon dioxide) or Aerosil Pharma 200, or Aerosil Pharma 300.

### <Test Example 3: Compatibility between natural product extract and additives>

For the development of an oral preparation with an improved dissolution rate and disintegrability of the natural product extract of the present invention, the compatibility between the processed ginseng extract as a natural product extract and additives was investigated.

Specifically, the additives were classified into diluents, solubilizers (dissolution aids), disintegrants, and lubricants, wherein lactose, starch, mannitol, dicalcium phosphate (CaHPO₄), and the like were used as diluents; poloxamers (Poloxamer 188 and Poloxamer 407), polyethylene glycol (PEG, PEG having a molecular weight of 200-6000), and the like were used as solubilizers; crospovidone, sodium croscamellose (Na-croscamellose), and the like may be used as disintegrants; and magnesium stearate (Mg-stearate), silicon dioxide (SiO₂), sodium stearyl fumarate (Na-stearyl fumarate), calcium silicon dioxide (CaSiO₂), glyceryl behenate, talc, and the like were used as lubricants.

The additives shown in Tables 4 and 5 below were mixed with the processed ginseng extract at a weight ratio of 1:1 (the amounts of additives mixed were significantly higher than the actual proportions of the additives in finished medicines) . After each of the mixtures was spread thin on a Petri dish, which was then closed with a lid, and the petri dish was sealed with a tape to ensure airtightness. The sealed Petri dish was stored for 3 months in a stability tester under long-term storage conditions of 25°C and relative humidity of 60% or accelerated storage conditions of 40°C and relative humidity of 75%, and the appearance was visually observed in the middle of the storage procedure. The results are shown in Tables 4 and 5 below.

**TABLE 4**

| Mixture | Long-term storage conditions (25°C, relative humidity of 60%) | | | | | | |
|---|---|---|---|---|---|---|---|
| | 3 days | 1 week | 2 weeks | 3 weeks | 4 weeks | 2 month s | 3 month s |
| Processed ginseng extract | - | - | - | - | - | - | - |
| Processed ginseng extract+Lactose | - | - | - | - | - | - | - |
| Processed ginseng extract+Starch | - | - | - | - | - | - | - |
| Processed ginseng extract+Mannitol | - | - | - | - | - | - | - |
| Processed ginseng extract+CaHPO₄ | - | - | - | - | - | - | - |
| Processed ginseng extract+Poloxamer* | - | - | - | - | - | - | - |
| Processed ginseng extract+PEG^{†} | - | - | - | - | - | - | - |
| Processed ginseng extract+Crospovidone | - | - | - | - | - | - | - |
| Processed ginseng extract+Na-croscarmellose | - | - | - | - | - | - | - |
| Processed ginseng extract+SiO₂^{‡} | - | - | - | - | - | - | - |
| Processed ginseng extract+Mg-stearate | - | - | - | - | - | - | - |
| Processed ginseng extract+Na-stearyl fumarate | - | - | - | - | - | - | - |
| Processed ginseng extract+CaSiO₂ | - | - | - | - | - | - | - |
| Processed ginseng extract+Glyceryl behenate | - | - | - | - | - | - | - |
| Processed ginseng extract+Talc | - | - | - | - | - | - | - |
| Poloxamer 407 or Poloxamer 188 | | | | | | | |
| ^{†} PEG with molecular weight of 200-6000 | | | | | | | |
| ^{‡} Aerosil Pharma 200 or Aerosil Pharma 300 | | | | | | | |

**TABLE 5**

| Mixture | Accelerated storage conditions (40°C, relative humidity of 75%) | | | | | | |
|---|---|---|---|---|---|---|---|
| | 3 days | 1 week | 2 weeks | 3 weeks | 4 weeks | 2 month s | 3 month s |
| Processed ginseng extract | - | Brown ing | Brown ing | Brown ing | Brown ing | Brown ing | Brown ing |
| Processed ginseng extract+Lactose | - | - | - | - | - | - | - |
| Processed ginseng extract+Starch | - | - | - | - | - | - | - |
| Processed ginseng extract+Mannitol | - | -- | - | - | - | - | - |
| Processed ginseng extract+CaHPO₄ | - | - | - | - | - | - | Agglo merat ing |
| Processed ginseng extract+Poloxamer* | - | - | - | - | - | Agglo merat ing | Agglo merat ing |
| Processed ginseng extract+PEG^{†} | - | - | - | - | - | - | - |
| Processed ginseng extract+Crospovidone | - | - | - | - | - | - | - |
| Processed ginseng extract+Na-croscarmellose | - | - | - | - | - | - | - |
| Processed ginseng extract+SiO₂^{‡} | - | - | - | - | - | - | - |
| Processed ginseng extract+Mg-stearate | - | - | - | - | - | Brown ing | Brown ing |
| Processed ginseng extract+Na-stearyl fumarate | - | - | - | - | - | Agglo merat ing | Agglo merat ing |
| Processed ginseng extract+CaSiO₂ | - | - | - | - | - | - | - |
| Processed ginseng extract+Glyceryl behenate | - | - | - | Agglo merat ing | Agglo merat ing | Agglo merat ing | Agglo merat ing |
| Processed ginseng extract+Talc | - | - | - | - | - | - | Agglo merat ing |
| Poloxamer 407 or Poloxamer 188 | | | | | | | |
| ^{†} PEG with molecular weight of 200-6000 | | | | | | | |
| ^{‡} Aerosil Pharma 200 or Aerosil Pharma 300 | | | | | | | |

Under the long-term storage conditions in Table 4 after sealing, no appearance change was observed even though the processed ginseng extract and each additive were mixed in excessive amounts.

However, under the accelerated storage conditions in Table 5, the processed ginseng extract alone underwent browning after 1 week of storage, and some mixtures also underwent agglomeration or browning. These results were considered to correspond to the change with temperature. In particular, the agglomeration occurring when the poloxamer was mixed as a solubilizer was considered to result from the use of the poloxamer excessively more than the amount used in the actual formulation of preparations despite the poloxamer is inevitably vulnerable to temperature due to the properties thereof. In addition, similar trends were also observed when magnesium stearate (Mg-stearate), sodium stearyl fumarate (Na-stearyl fumarate), glyceryl behenate, and talc are mixed, and these results were considered to result from the use of the additives with an amount being 3-10 times more than the amount used in the actual formulation of preparations, like in the poloxamer.

### <Test Example 4: Excipients for improving properties of natural product extract>

From the results of identifying solubilizers for improving the solubility and dispersibility of the processed ginseng extract as a natural product extract in Test Example 2, Gelucire 44/14, Transcutol P, Soluplus, Tween80, Cremophor EL, Cremophor RH40, poloxamers (Poloxamer 188, Poloxamer 407), PEGs (molecular weight of 200-6000), and Solutol HS15 were selected as solubilizers for improving solubility of the processed ginseng extract. As excipients, lactose, starch, mannitol, silicon dioxide (SiO₂), and calcium silicon dioxide (CaSiO₂) were selected to improve fluidity, and crospovidone and sodium croscarmellose (Na-croscarmellose) were selected to increase the rate of disintegration. In addition, dicalcium phosphate (CaHPO₄) and microcellulose, which are high-density excipients, were selected to make tablets or capsules with a certain size. As for all the excipients, commercially available excipients having a similar particle size to the processed ginseng extract were considered so as to ensure particle size uniformity and minimize the separation between powders.

The processed ginseng extract, solubilizers, and excipients were mixed according to the indications in Table 6 below. After 300 mg of the mixture was filled in each of No. 1 capsules, the filled capsules were visually observed for solubility and dispersibility through a disintegration test in a disintegrator containing a pH 1.2 buffer (dissolution medium) at 37°C. The results are shown in Table 6 below.

**TABLE 6**

| Mixture | | | | Result |
|---|---|---|---|---|
| Raw material | Excipient | Solubilizer | Mixing ratio (weight ratio) | |
| Processed ginseng extract | PEG (MW: 200-6000) | - | 1:1-2 | Wet within 15 min Dispersed within 20-30 min |
| | | | | PEG with lower molecular weight taking longer |
| | Poloxamer 188 | - | 1:1 | Wet within 5 min Dispersed within 15-20 min |
| | Poloxamer 407 | | | |
| | PEG400 | Gelucire 44/14 | 1:1:1 | Wet within 5 min |
| | | Poloxamer 188 | | Dispersed within 5 min |
| | | Poloxamer 407 | | |
| | | Solutol HS15 | | |
| | | Transcutol P | 1:1:1 | |
| | | Soluplus | | Wet within 30 min |
| | | Tween80 | | Dispersed within 30 min |
| | | Cremophor EL | | |
| | | Cremophor RH40 | | |
| | PEG6000 | Gelucire 44/14 | 1:1:1 | Wet within 5 min |
| | | Poloxamer 407 | | Dispersed within 15-20 min |
| | | Solutol HS15 | | |
| | PEG6000 | Poloxamer 407 | 1:0.1∼1:0.5-1 | Wet within 5 min |
| | | | | Dispersed within 5 min |
| | | | 1:0.1∼1:0.1 | Wet within ≤30 min |
| | | | | Dispersed within ≤30 min |
| | PEG6000 | Poloxamer 407 | 1:0.1∼0.5:0.1-1 | |
| | Lactose | | | Wet within 5 min |
| | Mannitol | | | Dispersed within 5 min |
| | SiO₂ | | | |
| | CaSiO₂ | | | |
| | PVP* | | | |
| | CaHPO₄ | Poloxamer 407 | 1:0.1∼0.5:0.1-1 | Wet within 15 min |
| | Isomalt* | | | Dispersed within 15 min |
| PVP(polyvinylpyrrolidone): used as Solubilizer | | | | |
| * Isomalt: used as diluent | | | | |

As shown in Table 6, the mixtures containing poloxamers, such as Poloxamer 188 and Poloxamer 407, Gelucire 44/14, Solutol HS15, PEG6000, lactose, mannitol, silicon dioxide (SiO₂), and the like, were wetted and dispersed within 5 minutes. Through the results of Test Examples 2 to 4, excipients for oral preparations with an improved dissolution and disintegration of the natural product extract of the present invention were established as shown in Table 7 below.

**TABLE 7**

| Formulation | |
|---|---|
| Constitution | Ingredient |
| Main ingredient | Natural product extract |
| Diluent | Lactose, CaHPO₄ |
| Solubilizer (dissolution aid) | Poloxamer, Polyethylene glycol (PEG) |
| Disintegrant | Crospovidone |
| Lubricant | SiO₂, Mg-stearate |
| Hard capsule | #1 |

### <Test Example 5: Establishment of conditions for oral preparation containing natural product extract>

Optimum conditions for oral preparations were established by investigating the dissolution rates of ginsenosides Rg3 and Rh2, which are active ingredients of the processed ginseng extract used as a natural product extract, according to each excipient, on the basis of the excipients in Table 7 for an oral preparation with an improved dissolution rate and disintegrability of the natural product extract of the present invention, identified in Test Example 4.

### Test example 5-1: Selection of type of lubricant

The dissolution rate of ginsenoside Rg3 or Rh2 was investigated for each oral preparation according to the type of silicon dioxide (SiO₂) among the lubricants in Table 7 above. As shown in Table 8 below, respective ingredients were mixed, and 310 mg of the mixtures were filled in No. 1 capsules, thereby manufacturing capsules of G34-1 to G34-4 in Table 8 below. The dissolution test of the manufactured capsules was performed according to the following conditions.
Apparatus: Apparatus 2 (paddle apparatus/Ph Eur.)
Paddle speed: 50 rpm
Dissolution medium: artificial gastric juice of pH 1.2 (containing 1% (w/v) Tween 80)
Volume of dissolution medium: 900 ml
Temperature of dissolution medium: 37±0.5°C
Capsule sinker: Copley 318 stainless steel

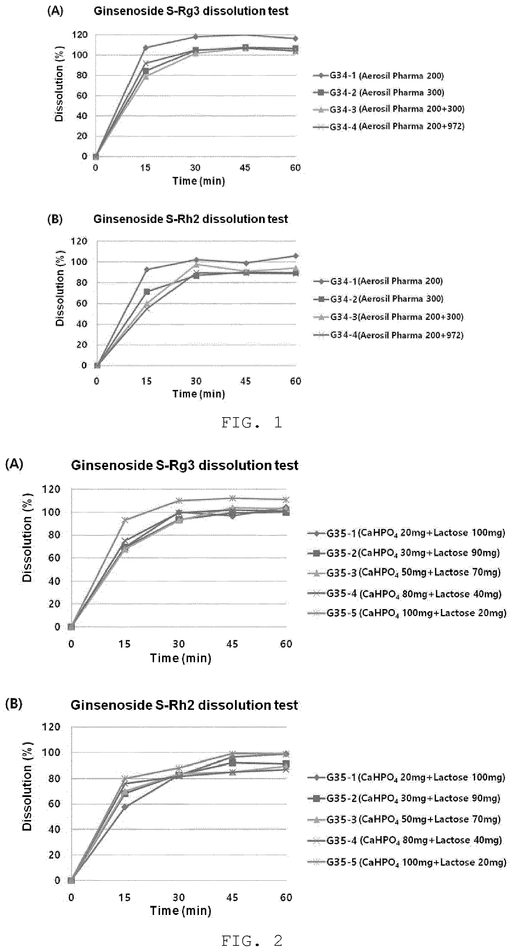

An aliquot of 10 ml was taken from the dissolution medium subjected to the dissolution test, and then filtered through a 0.45-µm regenerated cellulose syringe filter, thereby obtaining a filtrate. The dissolution rate of ginsenoside Rg3 or Rh2 was investigated by injecting 10 µl of the obtained filtrate into HPLC and performing the same method of HPLC as in Test Example 1. The results are shown in FIG. 1. The dissolution rate in the present invention was based on the standard of a dissolution of 75% or more within 45 minutes, and the dissolution rates within 15 minutes, 30 minutes, and 45 minutes were also compared according to the properties of the additives.

**TABLE 8**

| Formulation | | G34-1 | | G34-2 | | G34-3 | | G34-4 | |
|---|---|---|---|---|---|---|---|---|---|
| Consti tution | Ingredient | Base amount (mg) | Propor tion (%) | Base amount (mg) | Propor tion (%) | Base amount (mg) | Propor tion (%) | Base amount (mg) | Propor tion (%) |
| Main ingred ient | Processed ginseng extract | 100 | 32.3 | 100 | 32.3 | 100 | 32.3 | 100 | 32.3 |
| Solubi lizer | Poloxamer 407 | 60 | 19.4 | 60 | 19.4 | 60 | 19.4 | 60 | 19.4 |
| Lubric ant | SiO₂-1 (Aerosil Pharma 200) | 15 | 4.8 | - | - | 7.5 | 2.4 | 7.5 | 2.4 |
| | SiO₂-2 (Aerosil Pharma 300) | - | - | 15.0 | 4.8 | 7.5 | 2.4 | - | - |
| | SiO₂-3 | - | - | - | - | - | - | 7.5 | 2.4 |
| | (Aerosil Pharma972) | | | | | | | | |
| Diluen t | Lactose | 120 | 38.7 | 120 | 38.7 | 120 | 38.7 | 120 | 38.7 |
| Disint egrant | Crospovidone | 15 | 4.8 | 15 | 4.8 | 15 | 4.8 | 15 | 4.8 |
| Total | | 310 | 100.0 | 310 | 100.0 | 310 | 100.0 | 310 | 100.0 |

As shown in the results of identifying the dissolution rates of ginsenosides Rg3 (A) and Rh2 (B) according to the type of silicon dioxide (SiO₂) as a lubricant in FIG. 1, all the types of silicon dioxide were suitable for the standard regardless of the type of silicon dioxide when a dissolution rate of 75% or more within 45 minutes is based on the standard. However, as a result of comparing the 15-min dissolution rates, the dissolution rate of ginsenoside Rh2 was reduced by 20% or more in the mixing with the silicon dioxide Aerosil Pharma 972 (G34-4) compared with the mixing with Aerosil Pharma 200 (G34-1) or Aerosil Pharma 300 (G34-2), indicating that the other types of silicon dioxide excluding Aerosil Pharma 972 were suitable as lubricants, and it was identified that using silicon dioxide as a single ingredient was more suitable than using a mixture of silicon dioxide types.

It would also be preferable that the amount of the lubricant used was 15 mg/capsule or less considering the capsule size since the volume of the lubricant increased as the amount of a lubricant used increased.

### Test example 5-2; Content of dicalcium phosphate

The dissolution rate of ginsenoside Rg3 or Rh2 was investigated according to the content of dicalcium phosphate (CaHPO₄) (water-insoluble diluent) among the diluents in Table 7. As shown in Table 9 below, 310 mg of the mixtures containing respective ingredients were filled in No. 1 capsules, thereby manufacturing capsules of G35-1 to G35-5 in Table 9 below. The dissolution test and dissolution analysis of the manufactured capsules were carried out in the same manner as in Test Example 5-1. The results are shown in FIG. 2.

**TABLE 9**

| Formulation | | G35-1 | | G35-2 | | G35-3 | | G35-4 | | G35-5 | |
|---|---|---|---|---|---|---|---|---|---|---|---|
| Consti tution | Ingredient | Base amou nt (mg) | Prop orti on (%) | Base amou nt (mg) | Prop orti on (%) | Base amou nt (mg) | Prop orti on (%) | Base amou nt (mg) | Prop orti on (%) | Base amou nt (mg) | Prop orti on (%) |
| Main ingred ient | Processed ginseng extract | 100 | 32.3 | 100 | 32.3 | 100 | 32.3 | 100 | 32.3 | 100 | 32.3 |
| Solubi lizer | Poloxamer 407 | 60 | 19.4 | 60 | 19.4 | 60 | 19.4 | 60 | 19.4 | 60 | 19.4 |
| Lubric ant | SiO₂ (Aerosil Pharma 200) | 15 | 4.8 | 15 | 4.8 | 15 | 4.8 | 15 | 4.8 | 15 | 4.8 |
| Diluen t | CaHPO₄ | 20 | 6.5 | 30 | 9.7 | 50 | 16.1 | 80 | 25.8 | 100 | 32.3 |
| | Lactose | 100 | 32.3 | 90 | 29.0 | 70 | 22.6 | 40 | 12.9 | 20 | 6.5 |
| Disint egrant | Crospovidon e | 15 | 4.8 | 15 | 4.8 | 15 | 4.8 | 15 | 4.8 | 15 | 4.8 |
| Total | | 310 | 100. 0 | 310 | 100. 0 | 310 | 100. 0 | 310 | 100. 0 | 310 | 100. 0 |

As shown in FIG. 2, the dissolution rates were suitable for the standard at all the contents of dicalcium phosphate (CaHPO₄) when the dissolution rates of ginsenoside Rg3 (A) and Rh2 (B) were based on the standard of a dissolution rate of 75% or more within 45 minutes. Furthermore, as for the 15- and 30-min dissolution rates, the increasing content of dicalcium phosphate (CaHPO₄) advanced the disintegration time to increase the dissolution rates of ginsenosides Rg3 and Rh2. The dissolution rate of ginsenoside Rh2 was low until 30 minutes when 20 mg of dicalcium phosphate (CaHPO₄) was contained (G35-1).

It would be therefore preferable that the content of dicalcium phosphate was maintained at 30-100 mg/capsule considering the amounts mixed with other additives while increasing the initial dissolution rates of ginsenosides Rg3 and Rh2 and that the amount of a mixture obtained by mixing with another diluent lactose (water-soluble diluent) was adjusted to be 120 mg/capsule since the increasing content of a diluent increased the size of a capsule.

### Test example 5-3: Comparison of dissolution rates according to the presence or absence of lubricant and diluent

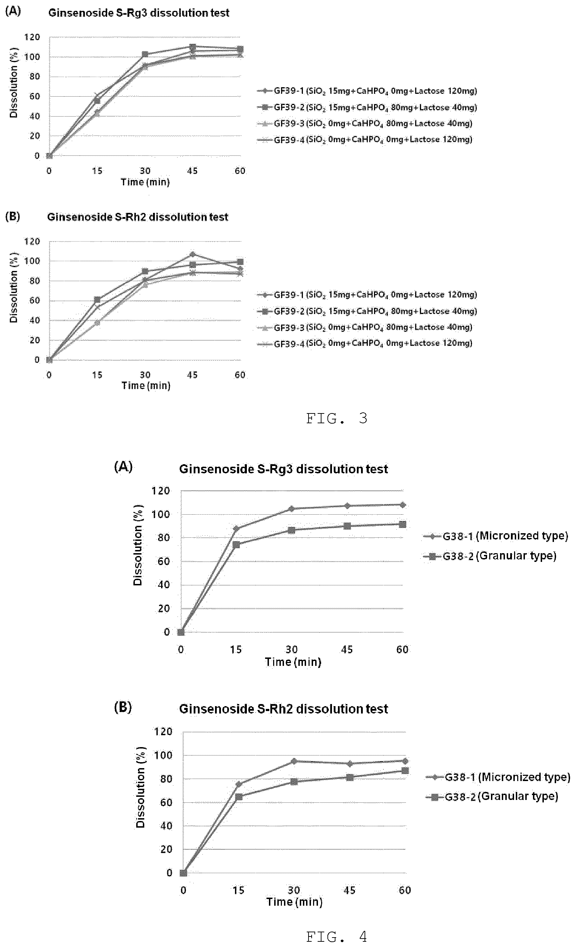

The dissolution rate of ginsenoside Rg3 or Rh2 was investigated according to the presence or absence of silicon dioxide (SiO₂) as a lubricant confirmed in Test Example 5-1 and dicalcium phosphate (CaHPO₄) and lactose as diluents confirmed in Test Example 5-2. As shown in Table 10 below, 310 mg or 295 mg of the mixtures containing respective ingredients were filled in No. 1 capsules, thereby manufacturing capsules of GF39-1 to G39-4. The dissolution test and dissolution rate analysis of the manufactured capsules were carried out in the same manner as in Test Example 5-1. The results are shown in FIG. 3.

**TABLE 10**

| Formulation | | GF39-1 | | GF39-2 | | GF39-3 | | GF39-4 | |
|---|---|---|---|---|---|---|---|---|---|
| Constit ution | Ingredient | Base amoun t (mg) | Propo rtion (%) | Base amoun t (mg) | Propo rtion (%) | Base amoun t (mg) | Propo rtion (%) | Base amoun t (mg) | Propo rtion (%) |
| Main ingredi ent | Processed ginseng extract | 100 | 32.3 | 100 | 32.3 | 100 | 33.9 | 100 | 33.9 |
| Solubil izer | Poloxamer 407 | 60 | 19.4 | 60 | 19.4 | 60 | 20.3 | 60 | 20.3 |
| Lubrica nt | SiO₂ (Aerosil Pharma 200) | 15 | 4.8 | 15 | 4.8 | - | - | - | - |
| Diluent | CaHPO₄ | - | - | 80 | 25.8 | 80 | 27.1 | - | - |
| | Lactose | 120 | 38.7 | 40 | 12.9 | 40 | 13.6 | 120 | 40.7 |
| Disinte grant | Crospovidone | 15 | 4.8 | 15 | 4.8 | 15 | 5.1 | 15 | 5.1 |
| Total | | 310 | 100.0 | 310 | 100.0 | 295 | 100.0 | 295 | 100.0 |

As shown in FIG. 3, the dissolution rates were suitable for the standard regardless of the mixing with silicon dioxide (SiO₂) as a lubricant and dicalcium phosphate (CaHPO₄) and lactose as diluents when the dissolution rates of ginsenoside Rg3 (A) and Rh2 (B) were based on the standard of a dissolution rate of 75% or more within 45 minutes. Furthermore, the mixture (GF39-2) obtained by mixing dicalcium phosphate (CaHPO₄) and lactose as diluents and silicon dioxide (SiO₂) showed superior dissolution rates over time compared with the mixture (GF39-1) obtained by mixing lactose and silicon dioxide (SiO₂) or the mixtures (GF39-3 and GF39-4) obtained by mixing only the diluents without silicon dioxide (SiO₂). Through these results, using a diluent and silicon dioxide (SiO₂) as a lubricant in mixture would be preferable for an oral preparation with an improved dissolution rate and disintegrability of the natural product extract of the present invention, and especially, using a water-insoluble diluent, such as dicalcium phosphate (CaHPO₄), as a diluent would be more preferable.

### Test example 5-4: Selection of type of poloxamer

Poloxamers, which are the solubilizers of Table 7, are classified into a granular type and a micronized type according to the size thereof. The dissolution rate of ginsenosides Rg3 or Rh2 was investigated according to the type of poloxamer. As shown in Table 11 below, 310 mg of the mixtures containing respective ingredients were filled in No. 1 capsules, thereby manufacturing capsules of G38-1 and G38-2 in Table 11 below. The dissolution test and dissolution analysis of the manufactured capsules were carried out in the same manner as in Test Example 5-1. The results are shown in FIG. 4.

**TABLE 11**

| Formulation | | G38-1 | | G38-2 | |
|---|---|---|---|---|---|
| Constitution | Ingredient | Base amount (mg) | Proportion (%) | Base amount (mg) | Proportion (%) |
| Main ingredient | Processed ginseng extract | 100 | 32.3 | 100 | 32.3 |
| Solubilizer | Poloxamer 407 (micronized type) | 60 | 19.4 | - | - |
| | Poloxamer 407 (granular type) | - | - | 60 | 19.4 |
| Lubricant | SiO₂ (Aerosil Pharma 200) | 15 | 4.8 | 15 | 4.8 |
| Diluent | CaHPO₄ | 80 | 25.8 | 80 | 25.8 |
| | Lactose | 40 | 12.9 | 40 | 12.9 |
| Disintegrant | Crospovidone | 15 | 4.8 | 15 | 4.8 |
| Total | | 310 | 100.0 | 310 | 100.0 |

As shown in FIG. 4, when the dissolution rates of ginsenoside Rg3 (A) and Rh2 (B) were based on the standard of a dissolution rate of 75% or more within 45 minutes, the dissolution rates were suitable for the standard for both the micronized type (G38-1) and granular type (G38-2) of poloxamers regardless of the type of poloxamer, showing dissolution rates of 75% or more within 15 minutes.

It could be seen through these results that the poloxamers improved the dissolution rates of both ginsenosides Rg3 and Rh2 regardless of the type of poloxamer and especially, the micronized type of poloxamer with a small particle size showed a higher effect of improving the dissolution rate of the processed ginseng extract.

### Test example 5-5; Content of poloxamer

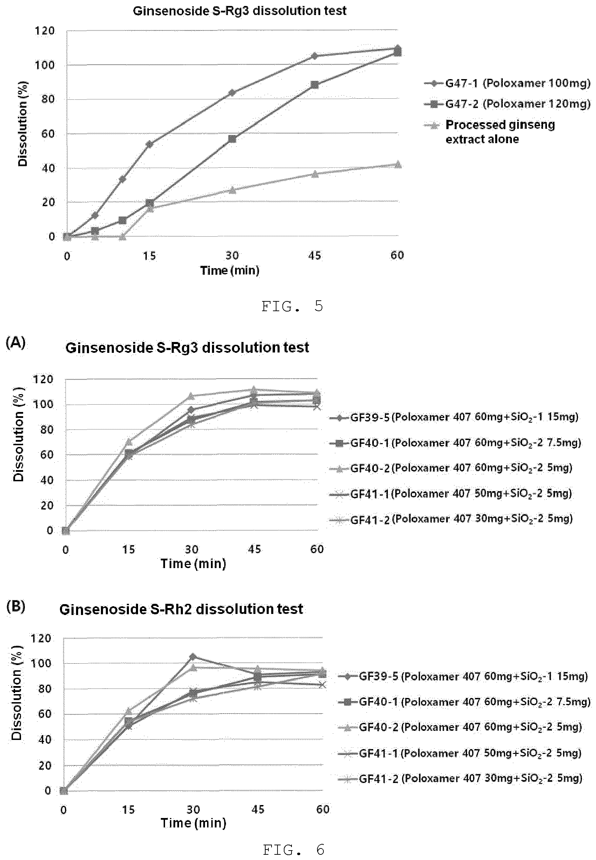

The dissolution rate of ginsenoside Rg3 was investigated according to the content of the poloxamer as a solubilizer in Table 7. As shown in Table 12 below, 310 mg of the mixtures containing respective ingredients were filled in No. 1 capsules, thereby manufacturing capsules of G47-1 and G47-2 in Table 12 below. The dissolution test and dissolution rate analysis of the manufactured capsules were carried out in the same manner as in Test Example 5-1. The results are shown in FIG. 5.

**TABLE 12**

| Formulation | | G47-1 | | G47-2 | |
|---|---|---|---|---|---|
| Constitution | Ingredient | Base amount (mg) | Proportion (%) | Base amount (mg) | Proportion (%) |
| Main ingredient | Processed ginseng extract | 100 | 32.3 | 100 | 32.3 |
| Solubilizer | Poloxamer 407 | 100 | 32.3 | 120 | 38.7 |
| Lubricant | SiO₂ (Aerosil Pharma 200) | 15 | 4.8 | 15 | 4.8 |
| Diluent | Lactose | 80 | 25.8 | 60 | 19.4 |
| Disintegrant | Crospovidone | 15 | 4.8 | 15 | 4.8 |
| Total | | 310 | 100.0 | 310 | 100.0 |

As shown in FIG. 5, when the dissolution rate of ginsenoside Rg3 was based on the standard of a dissolution rate of 75% or more within 45 minutes, the dissolution rate of ginsenoside Rg3 was up to 40% even after 60 minutes when the processed ginseng extract alone was filled, and both of the mixture (G47-1) of 100 mg of the poloxamer mixed with the processed ginseng extract or the mixture (G47-2) of 120 mg of the poloxamer mixed with the processed ginseng extract showed dissolution rates of 75% or more within 45 minutes, showing that those dissolution rates were suitable for the standard. In cases where the 15- and 30-min dissolution rates were compared, the mixture (G47-1) containing 100 mg of the poloxamer showed a higher dissolution rate than the mixture (G47-2) containing 120 mg of the poloxamer.

It was therefore determined that a poloxamer needs to be mixed for improvement of a dissolution rate and disintegrability of the natural product extract, and the content of the poloxamer is preferably used within 150 mg/capsule.

### Test example 5-6; Comparison of dissolution rates according to contents of poloxamer as solubilizer and silicon dioxide as lubricant and type of silicon dioxide

The dissolution rate of ginsenoside Rg3 or Rh2 was investigated according to the content of silicon dioxide (SiO₂) as a lubricant confirmed in Test Example 5-1, the content of the poloxamer as a solubilizer confirmed in Test Example 5-4, and the type of silicon dioxide. As shown in Table 13 below, the mixtures containing respective ingredients were filled in No. 1 capsules, thereby manufacturing capsules of GF39-5, GF40-1, GF40-2, GF41-1, and GF41-2 in Table 13 below. The dissolution test and dissolution analysis of the manufactured capsules were carried out in the same manner as in Test Example 5-1. The results are shown in FIG. 6.

**TABLE 13**

| Formulation | | GF39-5 | | GF40-1 | | GF40-2 | | GF41-1 | | GF41-2 | |
|---|---|---|---|---|---|---|---|---|---|---|---|
| Const ituti on | Ingredient | Base amou nt (mg) | Propo rtion (%) | Base amoun t (mg) | Propo rtion (%) | Base amoun t (mg) | Propo rtion (%) | Base amoun t (mg) | Propo rtion (%) | Base amoun t (mg) | Propo rtion (%) |
| Main ingre dient | Processed ginseng extract | 100 | 32.3 | 100 | 33.1 | 100 | 33.3 | 100 | 34.5 | 100 | 37.0 |
| Solub ilize r | Poloxamer 407 | 60 | 19.4 | 60 | 19.8 | 60 | 20.0 | 50 | 17.2 | 30 | 11.1 |
| Lubri cant | SiO₂-1 (Aerosil Pharma 200) | 15 | 4.8 | - | - | - | - | - | - | - | - |
| | SiO₂-2 (Aerosil Pharma 300) | - | - | 7.5 | 2.5 | 5 | 1.7 | 5 | 1.7 | 5 | 1.9 |
| Dilue nt | CaHPO₄ | 80 | 25.8 | 80 | 26.4 | 80 | 26.7 | 80 | 27.6 | 80 | 29.6 |
| | Lactose | 40 | 12.9 | 40 | 13.2 | 40 | 13.3 | 40 | 13.8 | 40 | 14.8 |
| Disin tegra nt | Crospovido ne | 15 | 4.8 | 15 | 5 | 15 | 5.0 | 15 | 5.2 | 15 | 5.6 |
| Total | | 310 | 100.0 | 302.5 | 100.0 | 300 | 100.0 | 290 | 100.0 | 270 | 100.0 |

As shown in FIG. 6, when the dissolution rates of ginsenosides Rg3 (A) and Rh2 (B) are based on the standard of a dissolution rate of 75% or more within 45 minutes, the dissolution rates were suitable for the standard regardless of the contents of the poloxamer and silicon dioxide (SiO₂) as a lubricant and the type of silicon dioxide.

However, as for 15- and 30-min dissolution rates of ginsenoside Rh2(B), the dissolution rates decreased as the content of the poloxamer decreased, and the dissolution rates depended on the content of silicon dioxide (SiO₂) in spite of the same content of the poloxamer.

It was therefore determined that the amounts of mixing of the poloxamer and silicon dioxide need to be adjusted in order to manufacture an oral preparation having an improved dissolution rate and disintegrability of the natural product extract, and the effect of further mixing with another additive needs to be considered.

### Test example 5-7: Comparison of dissolution rates according to addition of polyethylene glycol as solubilizer

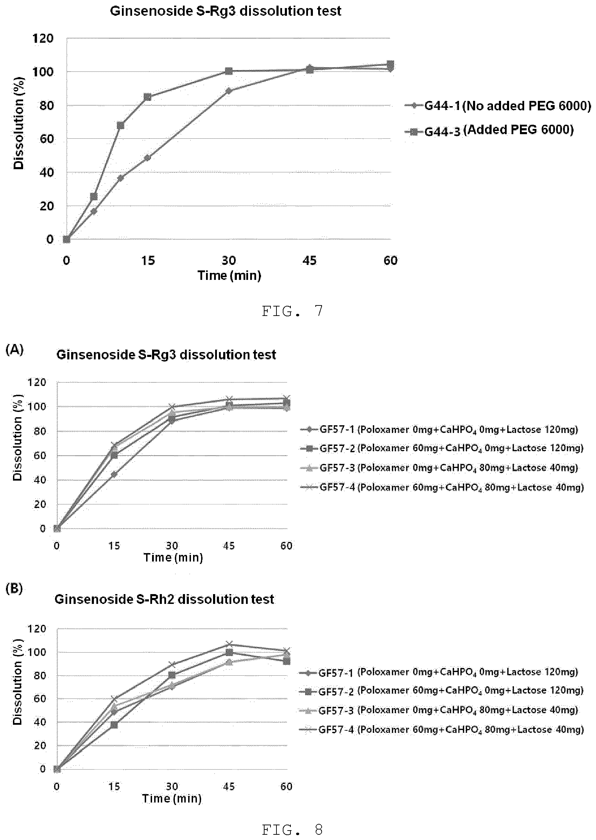

The dissolution rate of ginsenoside Rg3 or Rh2 was investigated according to the mixing with the poloxamer as a solubilizer confirmed in Test Examples 5-4 and 5-5 and polyethylene glycol (PEG) as another solubilizer in Table 7. As shown in Table 14 below, 305 mg or 317 mg of the mixtures containing respective ingredients were filled in No. 1 capsules, thereby manufacturing capsules of G44-1 and G44-3 in Table 14 below. The dissolution test and dissolution analysis of the manufactured capsules were carried out in the same manner as in Test Example 5-1. The results are shown in FIG. 7.

**TABLE 14**

| Formulation | | G44-1 | | G44-3 | |
|---|---|---|---|---|---|
| Constitution | Ingredient | Base amount (mg) | Proportion (%) | Base amount (mg) | Proportion (%) |
| Main ingredient | Processed ginseng extract | 100 | 32.8 | 100 | 31.5 |
| Solubilizer | Poloxamer 407 | 60 | 19.7 | 60 | 18.9 |
| | PEG6000 | - | - | 12 | 3.8 |
| Lubricant | SiO₂ (Aerosil Pharma 200) | 10 | 3.3 | 10 | 3.2 |
| Diluent | CaHPO₄ | 80 | 26.2 | 80 | 25.2 |
| | Lactose | 40 | 13.1 | 40 | 12.6 |
| Disintegrant | Crospovidone | 15 | 4.9 | 15 | 4.7 |
| Total | | 305 | 100.0 | 317 | 100.0 |

As shown in FIG. 7, the dissolution rates were suitable for the standard regardless of the further mixing with polyethylene glycol (PEG) when the dissolution rates of ginsenoside Rg3 (A) and Rh2 (B) were based on the standard of a dissolution rate of 75% or more within 45 minutes. However, as for the 15-min dissolution rate, the dissolution rate in the mixture (G44-1) not containing polyethylene glycol (PEG) was at least 20% lower than that in the mixture (G44-3) further containing polyethylene glycol (PEG).

Therefore, it would be preferable to use a poloxamer and polyethylene glycol in mixture, which are two types of solubilizers. The stability against heat may be reduced with an increasing amount of the solubilizer used, and thus 12 mg/capsule or less of polyethylene glycol and 60 mg/capsule or less of a poloxamer would be appropriate.

### Test example 5-8: Comparison of dissolution rates according to the presence of poloxamer and dicalcium phosphate

The dissolution rate of ginsenoside Rg3 or Rh2 was investigated according to the presence of a poloxamer as a solubilizer and dicalcium phosphate (CaHPO₄) as a water-insoluble diluent in Table 7. As shown in Table 15 below, the mixtures containing respective ingredients were filled in of No. 1 capsules, thereby manufacturing capsules in Table 15 below. The dissolution test and dissolution rate analysis of the manufactured capsules were carried out in the same manner as in Test Example 5-1. The results are shown in FIG. 8.

**TABLE 15**

| Formulation | | GF57-1 | | GF57-2 | | GF57-3 | | GF57-4 | |
|---|---|---|---|---|---|---|---|---|---|
| Consti tution | Ingredient | Base amount (mg) | Propor tion (%) | Base amount (mg) | Propor tion (%) | Base amount (mg) | Propor tion (%) | Base amount (mg) | Propor tion (%) |
| Main ingred ient | Processed ginseng extract | 100 | 40.0 | 100 | 32.3 | 100 | 40.0 | 100 | 32.3 |
| Solubi lizer | Poloxamer 407 | - | - | 60 | 19.4 | - | - | 60 | 19.4 |
| Lubric ant | SiO₂ (Aerosil Pharma 200) | 15 | 6.0 | 15 | 4.8 | 15 | 6.0 | 15 | 4.8 |
| Diluen t | CaHPO₄ | - | - | - | - | 80 | 32.0 | 80 | 25.8 |
| | Lactose | 120 | 48.0 | 120 | 38.7 | 40 | 16.0 | 40 | 12.9 |
| Disint egrant | Crospovidone | 15 | 6.0 | 15 | 4.8 | 15 | 6.0 | 15 | 4.8 |
| Total | | 250 | 100 | 310 | 100.0 | 250 | 100.0 | 310 | 100.0 |

As shown in FIG. 8, when the dissolution rates of ginsenosides Rg3 (A) and Rh2 (B) were based on the standard of a dissolution rate of 75% or more within 45 minutes, the dissolution rates were suitable for the standard regardless of whether the poloxamer and dicalcium phosphate (CaHPO₄) were contained. As for the 15- and 30-min dissolution rates, the dissolution rates were higher in the mixture (GF57-4) containing both the poloxamer and dicalcium phosphate (CaHPO₄) compared with the mixture (GF57-2) containing only the poloxamer or the mixture (GF57-3) containing only dicalcium phosphate (CaHPO₄), and especially, there was a greater difference in the dissolution rate of ginsenoside Rh2.

It was therefore seen that the mixing of a poloxamer and dicalcium phosphate as a water-insoluble diluent is important in order to manufacture an oral preparation having an improved dissolution rate and disintegrability of the natural product extract of the present invention.

### <Example 1: Establishment of prescription of final oral preparation containing natural product extract>

Based on the results confirmed in Test Examples 1 to 5, the prescription conditions of a final oral preparation of the present invention were selected as follows.
- Total weight: 280-340 mg as an appropriate dose for No. 1 capsule
- Main raw materials: 80-120 mg/capsule of natural product extract
- Excipients
   (1) Diluent: Considering the volume of a capsule, the dose of the diluent was delimited to 120 mg/capsule and the mixing ratio of dicalcium phosphate (CaHPO₄) as a water-insoluble diluent and lactose as a water-soluble diluent, which were generally used, was adjusted.
   (2) Solubilizer (dissolution aid): A poloxamer was used as a solubilizer. The poloxamer was mixed with polyethylene glycol having a molecular weight of 200-6000 since a fast dissolution tendency was shown when polyethylene glycol (PEG) was added to the poloxamer. Both granular and micronized types of poloxamers could be used, but the micronized type of poloxamers with a small particle size were preferably used.
   (3) Lubricant: Silicon dioxide was used, and magnesium stearate (within 5% (w/w) of the total weight) were usable in mix.
   (4) Disintegrant: A generally used dose (within 5% (w/w) of the total weight) was selected.
- The present invention is characterized in that a water-insoluble diluent such as dicalcium phosphate and a solubilizer are used in the manufacture of an oral preparation with an improved dissolution rate and disintegrability of a natural product extract (a water-soluble diluent and a disintegrant are generally used to improve the dissolution rate and disintegrability).

### <Example 2: Manufacture of final oral preparation containing natural product extract>

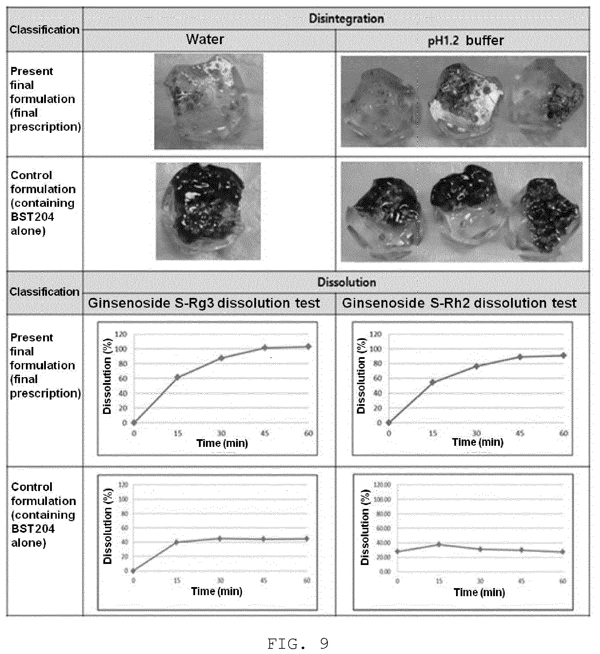

The mixtures were prepared according to the prescription in Table 16 below by using the processed ginseng extract as a natural product extract on the basis of the prescription of oral preparations established in Example 1 above, and then the mixtures were filled in No. 1 capsules, thereby manufacturing final oral preparations of the present invention. In addition, only the processed ginseng extract alone was filled in a capsule to manufacture an oral preparation for a control group. The dissolution rate and disintegrability of the manufactured capsules were compared according to the disintegration test method known in the Korean Pharmacopoeia. The dissolution rate was analyzed using the same method as the HPLC method of Test Example 5-1, and the results are shown in FIG. 9.

**TABLE 16**

| Oral preparation | | Capsule-1 | | Capsule-2 | | Capsule-3 | |
|---|---|---|---|---|---|---|---|
| Constitu tion | Ingredient | Base amount (mg) | Proport ion (%) | Base amount (mg) | Proporti on (%) | Base amount (mg) | Proporti on (%) |
| Main ingredie nt | Processed ginseng extract | 80 | 26.8 | 100 | 31.4 | 120 | 35.5 |
| Solubili zer | Poloxamer | 60 | 20.1 | 60 | 18.9 | 60 | 17.8 |
| | PEG6000 | 12 | 4.0 | 12 | 3.8 | 12 | 3.6 |
| Lubrican t | Silicon dioxide | 7.5 | 2.5 | 7.5 | 2.4 | 7.5 | 2.2 |
| | Magnesium stearate | 3.5 | 1.2 | 3.5 | 1.1 | 3.5 | 1.0 |
| Diluent | Dicalcium phosphate | 80 | 26.8 | 80 | 25.1 | 80 | 23.7 |
| | Lactose | 40 | 13.4 | 40 | 12.6 | 40 | 11.8 |
| Disinteg rant | Crospovidone | 15 | 5.0 | 15 | 4.7 | 15 | 4.4 |
| Total | | 298 | 100.0 | 318 | 100.0 | 338 | 100.0 |

As shown in FIG. 9, in the cases where it was based on the standard of a dilution of 75% or more within a dilution time of 45 minutes, the oral preparation of the control group containing the processed ginseng extract alone was agglomerated due to low solubility and wettability of the processed ginseng extract and showed dissolution rates of ginsenosides Rg3 and Rh2, but Capsule-1 to Capsule-3, which were oral preparations manufactured according to the final prescription of the present invention, showed improved disintegrability compared with the oral preparation of the control group, so that the disintegration was completed within 5 minutes and the dissolution rates of ginsenosides Rg3 and Rh2 were increased.

### <Example 3: Storage stability of final oral preparation containing natural product extract and change in dissolution rate according to storage>

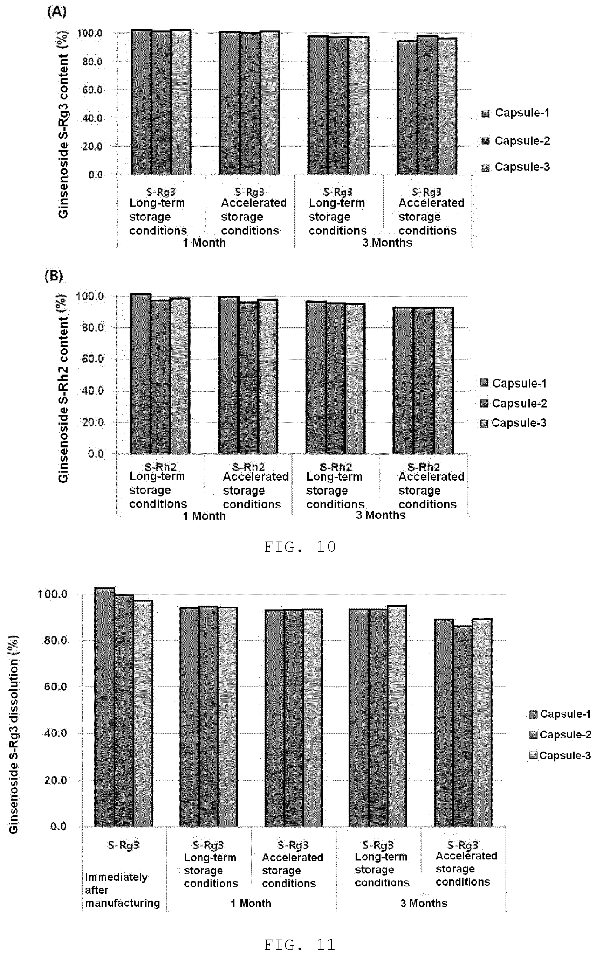

In order to identify the long-term stability and accelerated stability of Capsule-1 to Capsule-3 prepared in Example 2, the contents and dissolution rates of ginsenosides Rg3 and Rh2 (analysis of dissolution rate of ginsenoside Rg3 in a dissolution time of 45 minutes) in each capsule were investigated by the same method as in Test Example 5-1 while the manufactured capsules were stored at 25°C and relative humidity of 60% (long-term storage conditions) or at 40°C and relative humidity of 75% (accelerated storage conditions) in a stability tester for 3 months. The results are shown in FIGS. 10 and 11.

As can be seen from the results confirming the content changes of ginsenosides Rg3 (A) and Rh2 (B) according to the storage of Capsule-1 to Capsule-3, the contents of ginsenosides S-Rg3 and S-Rh2 were maintained at 100±5% in all the Capsule preparation 1 to Capsule preparation 3 while the capsule preparations were stored under long-term storage conditions and accelerated storage conditions for 3 months.

In addition, as can be seen from the results confirming the change in dissolution rate according to the storage period of Capsule-1 to Capsule-3 in FIG. 11, the dissolution rate of ginsenoside Rg3 was maintained at 80-90% and a dissolution rate of 75% or more within 45 minutes were maintained even when all the Capsule-1 to Capsule-3 were stored under long-term storage conditions and accelerated storage conditions for 3 months.

It could be therefore sufficiently predicted that the oral preparation containing the natural product extract of the present invention maintain the stability as well as dissolution rate of the natural product extract even when stored for a long period of time, thereby having an excellent dissolution rate and stability.

## Claims

1. An oral preparation containing a natural product extract and having an improved dissolution rate and disintegrability of the natural product extract, the oral preparation comprising a solubilizer, a water-insoluble diluent, a lubricant, and a disintegrant, wherein
i) the solubilizer is a mixture of a poloxamer and polyethylene glycol (PEG), the poloxamer and polyethylene glycol being mixed at a weight ratio of 1-10:1-10;
ii) the water-insoluble diluent is at least one selected from the group consisting of dicalcium phosphate, cellulose, and starch;
iii) the lubricant is at least one selected from the group consisting of silicon dioxide and magnesium stearate; and
iv) the disintegrant is at least one selected from the group consisting of crospovidone and sodium croscamellose, and
wherein the natural product extract is a processed ginseng extract or a ginseng extract.

2. The oral preparation of claim 1, wherein the oral preparation comprises, relative to 100 parts by weight of the natural product extract, 30-120 parts by weight of the solubilizer, 20-100 parts by weight of the water-insoluble diluent, 3-20 parts by weight of the lubricant, and 5-20 parts by weight of the disintegrant.

3. The oral preparation of claim 1, wherein the processed ginseng extract is prepared by:
(a) seeding *Aspergillus niger* into a medium composed of a ginseng powder and wheat bran;
(b) culturing the fungus in step (a);
(c) purifying the culture in step (b) through ultrafiltration;
(d) separating an enzyme from the purified product in step (c) ;
(e) adding the enzyme in step (d) to a ginseng powder, a red ginseng powder, a ginseng extract, or a red ginseng extract;
(f) fermenting the addition product in step (e);
(g) isolating the fermented product in step (f);
(h) concentrating the supernatant in step (g);
(i) reacting the concentrate in step (h) with at least one organic acid selected from the group consisting of acetic acid, lactic acid, citric acid, malic acid, and tartaric acid; and
(j) neutralizing, filtering, purifying, concentrating, and drying the reaction product in step (i).

4. The oral preparation of claim 1, wherein in the mixture of silicon dioxide and magnesium stearate, silicon dioxide and magnesium stearate are mixed at a weight ratio of 1-5:0.1-1.

5. The oral preparation of claim 1 or 2, further comprising a water-soluble diluent.

6. The oral preparation of claim 5, wherein the water-soluble diluent includes at least one selected from the group consisting of lactose, sucrose, mannitol, sorbitol, and dextrin.

7. The oral preparation of claim 5, wherein the water-soluble diluent is mixed with the water-insoluble diluent at a weight ratio of 1:0.2-5.

8. The oral preparation of claim 1 or 2, wherein the oral preparation comprises a poloxamer as the solubilizer and dicalcium phosphate as the water-insoluble diluent.

## Patentansprüche

1. Orales Präparat, das einen Naturproduktextrakt enthält und eine verbesserte Auflösungsrate und Desintegrierbarkeit des Naturproduktextrakts aufweist, wobei die orale Zubereitung einen Lösungsvermittler, ein wasserunlösliches Verdünnungsmittel, ein Gleitmittel und ein Desintegrationsmittel umfasst, wobei
i) der Lösungsvermittler eine Mischung aus einem Poloxamer und Polyethylenglykol (PEG) ist, wobei das Poloxamer und das Polyethylenglykol in einem Gewichtsverhältnis von 1-10:1-10 gemischt sind;
ii) das wasserunlösliche Verdünnungsmittel mindestens eines ist, das aus der Gruppe ausgewählt ist, die aus Dicalciumphosphat, Cellulose und Stärke besteht;
iii) das Gleitmittel mindestens eines ist, das aus der Gruppe ausgewählt ist, die aus Siliziumdioxid und Magnesiumstearat besteht; und
iv) das Desintegrationsmittel mindestens eines ist, das aus der Gruppe ausgewählt ist, die aus Crospovidon und Natriumcroscamellose besteht, und
wobei der Naturproduktextrakt ein verarbeiteter Ginseng-Extrakt oder ein Ginseng-Extrakt ist.

2. Orales Präparat nach Anspruch 1, wobei das orale Präparat, bezogen auf 100 Gewichtsteile des Naturproduktextrakts, 30-120 Gewichtsteile des Lösungsvermittlers, 20-100 Gewichtsteile des wasserunlöslichen Verdünnungsmittels, 3-20 Gewichtsteile des Gleitmittels und 5-20 Gewichtsteile des Desintegrationsmittels umfasst.

3. Orales Präparat nach Anspruch 1, wobei der verarbeitete Ginsengextrakt hergestellt wird durch:
(a) Aussäen von *Aspergillus niger* in ein Medium, das aus einem Ginsengpulver und Weizenkleie besteht;
(b) Kultivierung des Pilzes aus Schritt (a);
(c) Reinigen der Kultur aus Schritt (b) durch Ultrafiltration;
(d) Abtrennung eines Enzyms aus dem gereinigten Produkt aus Schritt (c);
(e) Zugabe des Enzyms in Schritt (d) zu einem Ginsengpulver, einem roten Ginsengpulver, einem Ginseng-Extrakt oder einem roten Ginseng-Extrakt;
(f) Fermentieren des Additionsprodukts aus Schritt (e);
(g) Isolieren des fermentierten Produkts aus Schritt (f) ;
(h) Konzentrieren des Überstandes aus Schritt (g);
(i) Umsetzen des Konzentrats aus Schritt (h) mit mindestens einer organischen Säure, ausgewählt aus der Gruppe bestehend aus Essigsäure, Milchsäure, Zitronensäure, Äpfelsäure und Weinsäure; und
(j) Neutralisieren, Filtrieren, Reinigen, Konzentrieren und Trocknen des Reaktionsprodukts aus Schritt (i).

4. Orales Präparat nach Anspruch 1, wobei in der Mischung aus Siliciumdioxid und Magnesiumstearat Siliciumdioxid und Magnesiumstearat in einem Gewichtsverhältnis von 1-5:0,1-1 gemischt sind.

5. Orales Präparat nach Anspruch 1 oder 2, das außerdem ein wasserlösliches Verdünnungsmittel umfasst.

6. Orales Präparat nach Anspruch 5, wobei das wasserlösliche Verdünnungsmittel mindestens eines aus der Gruppe bestehend aus Laktose, Saccharose, Mannit, Sorbit und Dextrin umfasst.

7. Orales Präparat nach Anspruch 5, wobei das wasserlösliche Verdünnungsmittel mit dem wasserunlöslichen Verdünnungsmittel in einem Gewichtsverhältnis von 1:0,2-5 gemischt ist.

8. Orales Präparat nach Anspruch 1 oder 2, wobei das orale Präparat ein Poloxamer als Lösungsvermittler und Dicalciumphosphat als wasserunlösliches Verdünnungsmittel umfasst.

## Revendications

1. Préparation orale contenant un extrait de produit naturel et ayant une vitesse de dissolution et une désintégrabilité améliorées de l'extrait de produit naturel, la préparation orale comprenant un solubilisant, un diluant insoluble dans l'eau, un lubrifiant et un désintégrant, dans laquelle
i) le solubilisant est un mélange de poloxamère et de polyéthylène glycol (PEG), le poloxamère et le polyéthylène glycol étant mélangés dans un rapport de poids de 1-10:1-10 ;
ii) le diluant insoluble dans l'eau est au moins un produit choisi dans le groupe constitué par le phosphate dicalcique, la cellulose et l'amidon ;
iii) le lubrifiant est au moins un produit choisi dans le groupe constitué par le dioxyde de silicium et le stéarate de magnésium ; et
iv) le désintégrant est au moins un produit choisi dans le groupe constitué de la crospovidone et de la croscamellose sodique, et
dans lequel l'extrait de produit naturel est un extrait de ginseng traité ou un extrait de ginseng.

2. La préparation orale de la revendication 1, dans laquelle la préparation orale comprend, par rapport à 100 parties en poids de l'extrait de produit naturel, 30-120 parties en poids du solubilisant, 20-100 parties en poids du diluant insoluble dans l'eau, 3-20 parties en poids du lubrifiant, et 5-20 parties en poids du désintégrant.

3. La préparation orale de la revendication 1, dans laquelle l'extrait de ginseng traité est préparé par :
(a) ensemencement d'*Aspergillus niger* dans un milieu composé de poudre de ginseng et de son de blé ;
(b) en cultivant le champignon de l'étape (a) ;
(c) purifier la culture de l'étape (b) par ultrafiltration ;
(d) séparer une enzyme du produit purifié de l'étape (c) ;
(e) ajout de l'enzyme de l'étape d) à une poudre de ginseng, à une poudre de ginseng rouge, à un extrait de ginseng ou à un extrait de ginseng rouge ;
(f) fermenter le produit d'addition de l'étape e) ;
(g) isoler le produit fermenté de l'étape f) ;
(h) concentration du surnageant de l'étape g) ;
(i) réaction du concentré de l'étape h) avec au moins un acide organique choisi dans le groupe constitué de l'acide acétique, de l'acide lactique, de l'acide citrique, de l'acide malique et de l'acide tartrique ; et
(j) neutralisation, filtrage, purification, concentration et séchage du produit de la réaction de l'étape i).

4. La préparation orale de la revendication 1, dans laquelle le dioxyde de silicium et le stéarate de magnésium sont mélangés dans un rapport de poids de 1-5:0,1-1.

5. La préparation orale de la revendication 1 ou 2, comprenant en outre un diluant soluble dans l'eau.

6. La préparation orale de la revendication 5, dans laquelle le diluant soluble dans l'eau comprend au moins un produit choisi dans le groupe constitué par le lactose, le saccharose, le mannitol, le sorbitol et la dextrine.

7. Préparation orale selon la revendication 5, dans laquelle le diluant soluble dans l'eau est mélangé au diluant insoluble dans l'eau dans un rapport de poids de 1:0,2-5.

8. La préparation orale de la revendication 1 ou 2, dans laquelle la préparation orale comprend un poloxamère comme solubilisant et du phosphate dicalcique comme diluant insoluble dans l'eau.
